(19) Europäisches Patentamt
European Patent Office
Office européen des brevets

(11) Publication number: **0 100 129**
**A1**

(12) # EUROPEAN PATENT APPLICATION

(21) Application number: **83201094.6**

(22) Date of filing: **27.07.83**

(51) Int. Cl.³: **C 08 G 18/02**, C 07 C 119/042, C 07 D 251/34

(30) Priority: **29.07.82 NL 8203025**

(43) Date of publication of application: **08.02.84 Bulletin 84/6**

(84) Designated Contracting States: **AT BE CH DE FR GB IT LI NL SE**

(71) Applicant: **DSM RESINS BV, Ceintuurbaan 5, NL-8022 AW Zwolle (NL)**

(72) Inventor: **Frisch, Kurt Charles, 17986 Park Lane, Grosse Ile Michigan 48138 (US)**
Inventor: **Wang, Sue-Ling, 17418 Warrington, Detroit Michigan 48221 (US)**

(74) Representative: **Hatzmann, Marinus Jan et al, OCTROOIBUREAU DSM P.O.Box 9, NL-6160 MA Geleen (NL)**

(54) **Process for preparing an oligomer of a diisocyanate.**

(57) The invention is directed to a process for preparing an oligomer of a diisocyanate, optionally in an inert solvent, in the presence of a suitable catalyst, followed by deactivation of the catalyst.

The invention is characterized in that a diisocyanate according to formula I of the formula sheet, where $R_1$ represents an alkyl group with one or more C atoms, a substituted or unsubstituted phenyl group or a group according to formula II of the formula sheet, where $R_1'$ and $R_1''$ represent alkyl groups with one or more C atoms, and where $R_2$, $R_3$ and $R_4$ are the same or different and are chosen from the group consisting of:
- alkyl groups with one or more C atoms
- alkoxy groups with one or more C atoms
- H,

is allowed to react at a temperature of between 10°C and 100°C in the presence of a metal salt of a $C_{2-20}$ carboxylic acid and/or of a $C_2$–$C_{20}$ alkanol in an amount of between 50 and $1000.10^{-6}$ mole/kg of polyisocyanate until the conversion desired has been reached, followed by deactivation of the reaction by addition of a deactivating agent from the group formed by inorganic acids, Lewis acids, and carboxylic acid halides in an amount at least stoichiometrically equivalent to the amount of catalyst.

EP 0 100 129 A1

# PROCESS FOR PREPARING AN OLIGOMER OF A DIISOCYANATE

The invention relates to a process for the oligomerization of an aliphatic diisocyanate.

It is known in the art that diisocyanates can be converted into oligomers thereof by reacting the diisocyanates, optionally in the presence of a catalyst. An important reason for this conversion of diisocyanates into the corresponding oligomers lies in the properties of the diisocyanate.

The fact is that diisocyanates are rather volatile and, moreover, toxic. By converting the diisocyanate into its oligomer a product is obtained which is easier to handle and, moreover, far less volatile.

One of the most frequently used aliphatic diisocyanates is 1,6 hexanediisocyanate. In the oligomerization of this compound problems are encountered.

If an effort is made to make an oligomer thereof in the usual manner, it will be found that, at a high degree of conversion, gelation of the reaction mixture will occur. It is possible, however, at a low degree of conversion for the non-reacted diisocyanate to be distilled off. Such a process is laborious, however, and very expensive energetically.

An alternative for this oligomerization is the formation of biuret. In this process a reaction product of two or more diisocyanate molecules is formed under the influence of water. A disadvantage of this process, however, is that it is difficult to reach a low free diisocyanate content. Moreover, this reaction is reversible, so that after some time the free diisocyanate content will rise.

As a consequence extra safety precautions must be taken in the processing of this product.

It is known that diisocyanates can be made to form oligomers by heating them in the absence of a catalyst or by letting them react at a lower temperature in the presence of a suitable catalyst. Known catalysts are i.a. Mannich bases, aziridines and phosphines. The reaction may be terminated by heating the reaction mixture to a temperature above the decomposition temperature of the heat-labile catalyst. Some drawbacks of the known methods are a tendency of the products towards discoloration, difficulties in controlling the reaction and, in case of deactivation by heating, difficulties in obtaining a consistent quality.

European patent application No. 56583 describes the oligomerization of polyisocyanates in the presence of a suitable trimerization catalyst followed by deactivation thereof.

It is an object of the invention to provide a process for preparing an improved oligomer of a diisocyanate.

The process according to the invention is characterized in that a diisocyanate according to formula I of the formula sheet where $R_1$ represents an alkyl group with one or more C atoms, a substituted or unsubstituted phenyl group or a group according to formula II of the formula sheet, where $R_1'$ and $R_1''$ represent alkyl groups with one or more C atoms, and where $R_2$, $R_3$ and $R_4$ are the same or different and are chosen from the group consisting of:

- alkyl groups with one or more C atoms
- alkoxy groups with one or more C atoms
- H,

is allowed to react at a temperature of between 10 °C and 100 °C in the presence of a metal salt of a $C_{2-20}$ carboxylic acid and/or of a $C_1 - C_{20}$ alkanol in an amount of between 50 and $1000.10^{-6}$ mole/kg of polyisocyanate until the conversion desired has been reached, followed by deactivation of the reaction by addition of a deactivating agent from the group formed by inorganic acids, Lewis acids, and carboxylic acid halides in an amount at least stoichiometrically equivalent to the amount of catalyst.

The advantages of the process are that it can be carried out using inexpensive catalysts and deactivating agents, that the reaction can be closely controlled so that a consistent quality is obtained, that the product has a good storage stability and little or no tendency towards discoloration and that it has a good reactivity. The process is applicable to aromatic as well as aliphatic polyisocyanates.

The catalysts used are preferably alkali or earth alkali metal salts of carboxylic acids containing from 2 to 20 carbon atoms and/or of a $C_1$ - $C_{20}$ alkanol. The sodium salts would seem to give the best results. The carboxylic acids may be monofunctional or polyfunctional, aliphatic, cycloaliphatic or aromatic, saturated as well as unsaturated. Although the salts of dicarboxylic acids such as isophthalic acid, sebacic acid, adipic acid or fumaric acid can be used, the salts of the monocarboxylic acids are generally preferred due to a lower price and easier handling. Suitable are the salts of i.a. acetic acid, butyric acid, hexanoic acid, octanoic acid, stearic acid, acrylic acid, oleic acid, benzoic acid, m.toluic acid, anisic acid. Although the metal could be lithium or cesium, the readily available salts of sodium or potassium will be used in most cases, with a slight preference for sodium salts. Generally the best results are obtained by using aromatic carboxylic acids, particularly benzoates.
The amount of carboxylate salt may range between 50 and $1000.10^{-6}$ mole/kg of diisocyanate and preferably between 100 and 500 mole/kg, and more specifically between 200 and $400.10^{-6}$ mole/kg.

It is also possible to use an alkali metal alkanolate, such as sodium methoxylate.

Optionally a cocatalyst can be used together with the alkali metal salts of a carboxylic acid or an alkanol. Suitable cocatalysts are aliphatic or aromatic hydroxyl-compounds, such as phenol or hexanol-1.

The reaction may be carried out at temperatures of between 10 °C and 100 °C. At temperatures below room temperature the reaction will generally proceed too slowly, while at temperatures above 100 °C the danger of a too fast reaction, leading to gelation, is present. In general, a temperature of between 20 °C and 50 °C is preferred.

The reaction time will in general range between 0.5 and 24 hours, depending on the reactants, the temperature and the degree of conversion which is desired. Longer reaction times can be used, however. The reaction can be continued until the desired conversion has been reached. In general, it will be desired to reduce the amount of free diisocyanates to a value of less than 1 % by weight, based on the weight of the solution, and when possible to less than 0,5 % by wt. If the free isocyanate content of the reaction mixture is deemed to high, it can be also reduced by removal of diisocyanate after deactivation of the reaction. This removal can be effected by distillation at a reduced pressure.

The reaction will be terminated by the addition of a deactivation agent which will react with the alkali carboxylate and convert it to a catalytically inactive alkali salt. The deactivating agent will thus be added in an amount which is at least stoichiometrically equivalent to the amount of catalyst. A too large excess of deactivating agent might interfere with subsequent reactions or have a negative influence on the physical properties of the polyurethane end products. An excess of up to 100 mole % could be used and preferably the excess will be 5 to 25 mole %. Suitable deactivating agents comprise inorganic acids, Lewis acids, carboxylic acid halides and carbamoyl chlorides. The carboxylic compounds will be derived from $C_{2-20}$ carboxylic acids, and the halide is generally a chloride. Some suitable compounds are hydrochloric acid, boron trifluoride, aluminium trichloride, acetyl chloride, butyryl chloride, adipoyl chloride, myristyl chloride, benzoyl chloride and naphtoyl chloride. The aromatic carboxylic acid chlorides are preferred, since they are effective deactivating agents which improve the stability and colour of the final product.

Of the diisocyanate, $R_1$ is preferably an alkyl group and $R_2$, $R_3$ and $R_4$ are hydrogen atoms. More particularly, a methyl group is used for $R_1$. In that case 1,5 hexanediisocyanate is obtained.

In another preferred mode of realizing the invention $R_1$ and $R_2$ jointly form a cycloaliphatic ring. This ring preferably contains 5-12 C-atoms, as indicated in Formula III.

The reaction can be carried out in the absence of a solvent, in cases of liquid polyisocyanates, or with the polyisocyanate suspended in an inert solvent. It is, however, preferred to dissolve the polyisocyanate in an inert solvent, as the reaction is easier to control in this way. Suitable solvents comprise those known in polyurethane chemistry such as aromatic hydrocarbons, e.g. toluene or xylene, lower alkyl esters of carboxylic acids, e.g. ethylacetate, butylacetate, methyl propionate, ethers e.g. dioxane and tetrahydrofurane, sulfoxy compounds as dimethylsulfoxide, and ethers and esters of polyethylene glycols, e.g. cellosolve acetate. The concentration of the polyisocyanate generally is between 30 and 70 % by weight.

The oligomer may be used for commercial purposes as a solution in a solvent. The solid content of the oligomer solution then preferably amounts to 5-75 % (wt), more particularly 35-60 % (wt).

After the desired degree of conversion and the desired isocyanate (NCO) content have been reached, the reaction can be stopped in a manner known per se, for instance by the addition of an acid chloride, such as benzoylchloride.

In the preparation of the oligomer the diisocyanate further defined in the claims is started from. The diisocyanate can, for instance, be prepared from the corresponding diamine by phosgenation, or in another manner known in the art.

The diamine can be obtained in the various manners known in the art. An effective manner is the process described in Chem. Ber. 99 (10) 3387-9 (1966), for, among other things 1,5 diaminohexane. An alternative manner is the hydrogenation, in ammoniacal conditions, of the corresponding ketonitrile compound.

Preferably the oligomer does not contain more than 2.0 % (wt) free diisocyanate, more particularly not more than 0.5 % (wt).

The content of NCO groups in the oligomer is preferably between 10 and 25 % (wt). The weight average molecular weight can preferably be between 500 and 5000, more particularly between 500 and 1500.

The most suitable values for NCO content and molecular weight may vary according to application. The most suitable value according to application can be determined in a simple manner by the person skilled in the art.

Surprisingly, it has been found that the oligomer according to the invention has a number of very favourable properties.

An important property first manifests itself already in the preparation of the oligomer. The fact is that it has been found that the oligomer according to the invention, unlike for instance the oligomers of 1,6 hexane-diisocyanate, can be prepared in a very simple manner and with a high degree of conversion (> 99 %).

While applying an oligomerization process according to the invention the oligomer is obtained from the corresponding diisocyanate with a high yield and with high selectivity. Without much trouble a product can be made having a free diisocyanate content of at most 0.3 % (wt) without free diisocyanate having to be removed or without gel building.

Other advantages of the oligomer prepared according to the invention are in its use in, for instance, paints and lacquers. It has been found that the use of the oligomer results in a harder coat of paint having an equally good or even better resistance against external effects compared with, for instance, a coat of paint based on 1,6 hexanediisocyanate. Moreover, a paint or lacquer based on the oligomer according to the invention has a longer pot life.

The oligomer according to the invention can in principle be used for all diisocyanate applications known in the art. Of particular importance, however, are the higher grade uses of aliphatic diisocyanates, such as industrial wood lacquers, car repair lacquers, airplane lacquers, leather lacquers, textile coatings and the like.

The invention will now be elucidated by means of a few non-restrictive examples.

Example 1

To a 50 wt.% solution of 1,5 hexanediisocyanate in butylacetate 4 wt.% (w.r.t. diisocyanate) of a 1/1 (wt.)mixture of sodium-methoxylate and phenol was added at room temperature. Within 3 minutes a conversion of diisocyanate to oligomer of more than 99.5 % was obtained.

Example 2

In the same way as in 1, 1 wt.% of a 1/1 wt. mixture of sodium-methoxylate was used.
After a diisocyanate content of less than 0.2 wt.% had been reached, the reaction was stopped with benzoylchloride.

Example 3

Example 2 was repeated, using 1 wt.% of sodiumoctoate. Comparable results were obtained.

## CLAIMS

1. Process for preparing an oligomer of a diisocyanate, optionally in an inert solvent, in the presence of a suitable catalyst, followed by deactivation of the catalyst,
characterized in that a diisocyanate according to formula I of the formula sheet, where $R_1$ represents an alkyl group with one or more C atoms, a substituted or unsubstituted phenyl group or a group according to formula II of the formula sheet, where $R_1'$ and $R_1''$ represent alkyl groups with one or more C atoms, and where $R_2$, $R_3$ and $R_4$ are the same or different and are chosen from the group consisting of:
- alkyl groups with one or more C atoms
- alkoxy groups with one or more C atoms
- H,

is allowed to react at a temperature of between 10 °C and 100 °C in the presence of a metal salt of a $C_2$-$C_{20}$ carboxylic acid and/or of a $C_1$ - $C_{20}$ alkanol in an amount of between 50 and $1000.10^{-6}$ mole/kg of polyisocyanate until the conversion desired has been reached, followed by deactivation of the reaction by addition of a deactivating agent from the group of inorganic acids, Lewis acids, and carboxylic acid halides in an amount at least stoichiometrically equivalent to the amount of catalyst.
2. Process according to claim 1, characterized in that the amount of catalyst used is between 200 and $400.10^{-6}$ moles/kg of polyisocyanate.
3. Process according to claim 1 or 2, characterized in that the catalyst is sodium benzoate.
4. Process according to claim 1, characterized in that the deactivating agent is used in an amount exceeding the stoichiometrically required amount by 5 to 25 %.
5. Process according to claim 1 or 4, characterized in that the deactivating agent is benzoyl chloride.
6. Process according to claims 1-5, characterized in that a secondary catalyst is used in combination with the carboxylic acid and/or alkanol compound.

7. Process according to claim 6, characterized in that an aromatic or aliphatic compound containing a hydroxyl group is used.
8. Process according to claim 7, characterized in that phenol is used.
9. Process according to claims 1-8, characterized in that the oligomerization is allowed to proceed until the amount of unreacted polyisocyanate is less than 0,5 wt% calculated on the reaction mixture.
10. Process according to claims 1-9, characterized in that $R_1$ represents an alkyl group and $R_2$, $R_3$ and $R_4$ represent hydrogen atoms.
11. Process according to claims 1-10, characterized in that $R_1$ is a methyl group.
12. Process according to claims 1-9, characterized in that $R_1$ and $R_2$ form a cycloaliphatic ring with 5-12 C atoms.
13. Process according to claim 12, characterized in that the cycloaliphatic ring contains 5, 6 or 12 C atoms.
14. Process substantially as described and elucidated with respect to the examples.

Formula page

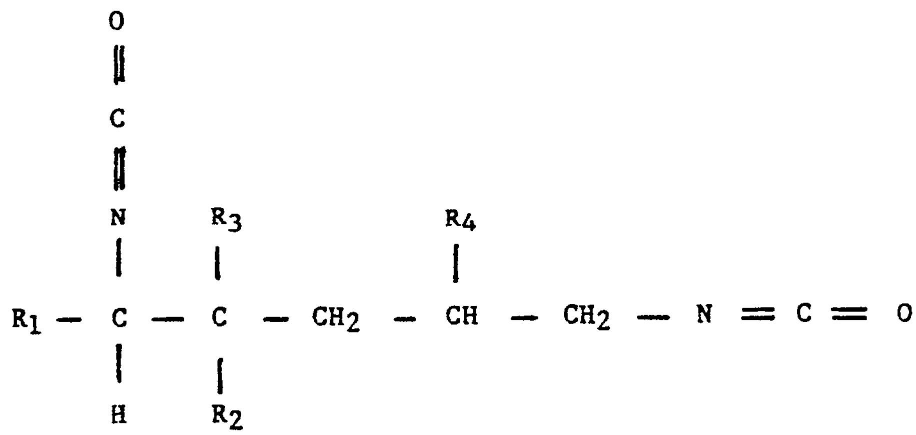

Formula 1

$$R_1'' - O - R_1'$$

Formula 2

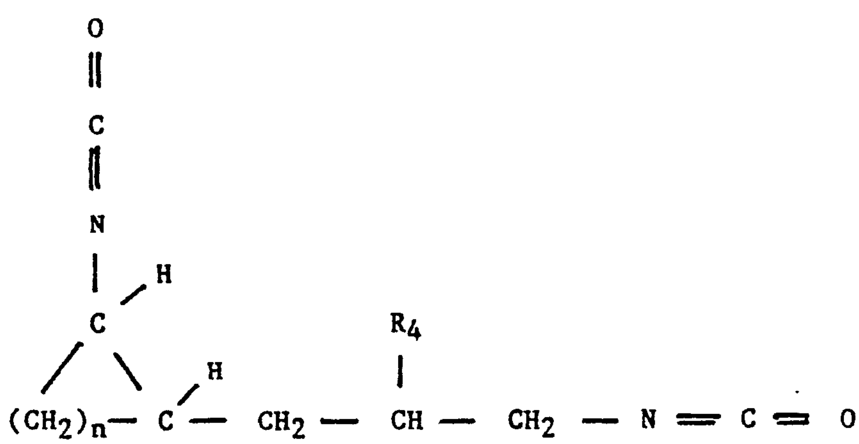

Formula 3

European Patent Office

# EUROPEAN SEARCH REPORT

0100129
Application number

EP 83 20 1094

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X,P | EP-A-0 077 104 (STAMICARBON)<br>* Page 8, claims 1-5,9; page 2, lines 19-33; page 3, lines 4-7 *<br>--- | 1,10-14 | C 08 G 18/02<br>C 07 C 119/042<br>C 07 D 251/34 |
| A | FR-A-1 190 065 (I.C.I.)<br>* Page 6, abstract points A1-A3; page 1, left-hand column, last paragraph - right-hand column, last paragraph; page 2, left-hand column, paragraph 3 *<br>--- | 1 | |
| A | GB-A- 920 080 (I.C.I.)<br>* Page 3, claims 1,4; page 1, lines 41-80; page 2, lines 5-20 *<br>--- | 1,3,6-8 | |
| | | | TECHNICAL FIELDS SEARCHED (Int. Cl. ³) |
| A | GB-A- 949 253 (I.C.I.)<br>* Page 3, claims 1,5; page 2, lines 5-25 *<br>--- | 1 | |
| A | EP-A-0 056 583 (BASF WYANDOTTE)<br>* Page 31, claim 1; page 9, lines 1-7; page 10, lines 4-14 *<br>----- | 1,5 | C 08 G<br>C 07 C<br>C 07 D |

The present search report has been drawn up for all claims

| Place of search | Date of completion of the search | Examiner |
|---|---|---|
| THE HAGUE | 02-11-1983 | VAN PUYMBROECK M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82